# EUROPEAN PATENT SPECIFICATION

(11) **EP 3 827 821 B1**
(45) Date of publication and mention of the grant of the patent: **26.10.2022**
(21) Application number: 20210104.4
(22) Date of filing: 26.11.2020
(51) Int. Cl.: A61K 31/19, A61K 31/43, A61P 25/08

(54) **USE OF PHARMACEUTICAL COMPOSITION TO TREAT EPILEPSY SEIZURE AND/OR TO TREAT EPILEPSY ASSOCIATED MOTOR SYMPTOM AND COGNITIVE IMPAIRMENT**
VERWENDUNG EINER PHARMAZEUTISCHEN ZUSAMMENSETZUNG ZUR BEHANDLUNG VON EPILEPSIEANFÄLLEN UND/ODER ZUR BEHANDLUNG VON MIT EPILEPSIE VERBUNDENEN MOTORISCHEN SYMPTOMEN UND KOGNITIVEN BEEINTRÄCHTIGUNGEN
UTILISATION D'UNE COMPOSITION PHARMACEUTIQUE POUR TRAITER UNE CRISE D'ÉPILEPSIE ET/OU TRAITER UN SYMPTÔME MOTEUR ET UN TROUBLE COGNITIF ASSOCIÉ À L'ÉPILEPSIE

(30) Priority: 26.11.2019 TW 108142968
(43) Date of publication of application: 02.06.2021
(73) Proprietor: Arthur Wisdom Technology Consultants Co., Ltd., Taichung City 408032 (TW)
(72) Inventor: Ho, Ying-Jui, Taichung City (TW); Chen, Jian-Horng, Taichung City (TW)
(74) Representative: Lang, Christian

(56) References cited:
- US-A1- 2010 255 099
- RAWLS S M ET AL: "@b-lactamase inhibitors display anti-seizure properties in an invertebrate assay", NEUROSCIENCE, NEW YORK, NY, US, vol. 169, no. 4, 15 September 2010 (2010-09-15), pages 1800-1804, XP027197738, ISSN: 0306-4522 [retrieved on 2010-06-23]
- MACIEJ GASIOR ET AL: "Clavulanic acid does not affect convulsions in acute seizure tests in mice", JOURNAL OF NEURAL TRANSMISSION, SPRINGER-VERLAG, VI, vol. 119, no. 1, 3 June 2011 (2011-06-03), pages 1-6, XP019996821, ISSN: 1435-1463, DOI: 10.1007/S00702-011-0662-1

## Description

### BACKGROUND OF THE INVENTION

### 1. Field of the Invention

The present invention generally relates to a use of a pharmaceutical composition including clavulanic acid and valproic acid and, more particularly, to a use of a pharmaceutical composition to treat epilepsy seizure and/or to treat epilepsy-associated motor symptom and cognitive impairment.

### 2. Description of the Related Art

Epilepsy is a neurological medical condition characterized by seizure (epileptic seizure). Many causes will result in epilepsy. Abnormal electrical activity in brain of epilepsy patients will cause seizure.

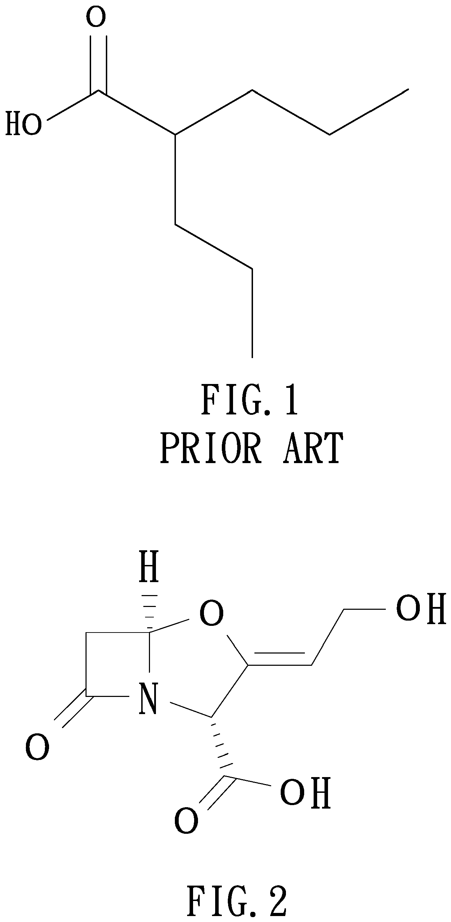

Valproic acid with a chemical structure shown in FIG. 1 is the first-line treatment among the conventional anticonvulsant drugs. Valproic acid can be used to prevent from seizure, but about 30% of epilepsy patients cannot be effectively controlled even if treated with valproic acid. Moreover, valproic acid not only causes side effects including nausea, vomiting, sleepiness and dry mouth, but also affects liver function and renal tubular injury. Therefore, epilepsy patients taking valproic acid should regularly monitor their liver and renal functions, causing inconvenience in their lives.

Besides seizure, epilepsy also causes neuron lesion or neuronal cell death in brain, leading to motor symptoms and cognitive impairments. As an example, epilepsy affects not only motor coordination and balance, but also recognition and memory; and therefore, epilepsy patient has reduced quality of life (QoL). However, the conventional anticonvulsant drug such as valproic acid cannot improve neuron lesion caused by epilepsy, but increase γ-aminobutyric acid (GABA), an inhibitory neurotransmitter, level in brain and spinal cord, inhibiting neuronal, behavioral and recognition functions. That is, the administration of the conventional anticonvulsant drug (valproic acid) cannot improve the behavioral symptoms, but will make the symptoms worse.

In addition, the conventional anticonvulsant drug (valproic acid) should be administered continuously. If the epilepsy patients have poor medication compliance and do not correctly follow the medical advice, the epilepsy patients have increased risk of seizure or status epilepticus. In light of this, the conventional anticonvulsant drug should be improved.

### SUMMARY OF THE INVENTION

It is therefore an objective of the present invention to provide a pharmaceutical composition for treatment of epilepsy seizure and/or for treatment of epilepsy-associated motor symptom and cognitive impairment.

One embodiment of the present invention discloses a pharmaceutical composition for use in the treatment of epilepsy seizure comprising clavulanic acid and valproic acid. Alternatively, another embodiment of the present invention discloses a pharmaceutical composition for use in the treatment of epilepsy-associated motor symptom and cognitive impairment comprising clavulanic acid and valproic acid.

Accordingly, by the synergistic effect of clavulanic acid and valproic acid, the pharmaceutical composition of the present invention can be used to prevent from seizure, to decrease seizure intensity and to trigger neuroregeneration in hippocampal dentate gyrus area, treating seizure and epilepsy-associated motor symptom and cognitive impairment, such as the motor coordination and balance problems, the cognitive impairment on object recognition ability and the cognitive impairment on memory.

Moreover, the co-administration with clavulanic acid can reduce the dosage of valproic acid needed, therefore can be rapidly and effectively metabolized by the metabolic organs such as liver and kidney, preventing valproic acid from accumulation in the organism. Furthermore, the reduced dosage of valproic acid can also decrease the burden to the metabolic organs such as liver and kidney, as well as diminish the risk of side effects.

In another preferred form shown, clavulanic acid and valproic acid are to be co-administrated to a subject in need thereof.

In another preferred form shown, clavulanic acid can be to be administrated to the subject in a dosage of 0.016-10 mg/kg/day. Preferably, clavulanic acid can be to be administrated to the subject in the dosage of 0.016-4.99 mg/kg/day. In addition, valproic acid can be to be administrated to the subject in a dosage of 0.8-30 mg/kg/day. Preferably, valproic acid can be to be administrated to the subject in the dosage of 0.8-19.99 mg/kg/day. As such, seizure, as well as epilepsy-associated motor symptom and cognitive impairment, is effectively treated.

In another preferred form shown, the pharmaceutical composition can be continuously or intermittently administrated to the subject. As such, seizure, as well as epilepsy-associated motor symptom and cognitive impairment, is effectively treated.

### BRIEF DESCRIPTION OF THE DRAWINGS

The present invention will become more fully understood from the detailed description given hereinafter and the accompanying drawings which are given by way of illustration only, and thus are not limitative of the present invention, and wherein:
FIG. 1 depicts a diagram illustrating the chemical structure of valproic acid.
FIG. 2 depicts a diagram illustrating the chemical structure of clavulanic acid.
FIG. 3 depicts a line chart illustrating the seizure intensity of the rats of groups A0-A5 in trial (B).
FIG. 4a depicts a schematic diagram illustrating the rat R under the test rides on an accelerating rotarod W in trial (C).
FIG. 4b depicts a schematic diagram illustrating the rat R under the test falls from the accelerating rotarod W in trial (C).
FIG. 5 depicts a bar chart illustrating the riding time of the rats of groups A0-A5 in trial (C).
FIG. 6a depicts a schematic diagram illustrating an open box used for the exposure session in trial (D).
FIG. 6b depicts a schematic diagram illustrating the open box used for a test session in trial (D).
FIG. 7 depicts a bar chart illustrating the percentage of time exploring object of the rats of groups A0-A5 in trial (D).
FIG. 8a depicts a schematic diagram illustrating a shuttle box used for an exploration session in trial (E). The rat R under the test is allowed to move freely into a light chamber C1.
FIG. 8b depicts a schematic diagram illustrating the shuttle box used for a learning session in trial (E). The rat R under the test is placed in the light chamber C1.
FIG. 8c depicts another schematic diagram illustrating the shuttle box used for the learning session in trial (E). The rat R under the test enters a dark chamber C2 due to photophobia.
FIG. 8d depicts yet another schematic diagram illustrating the shuttle box used for the learning session in trial (E). The rat R under the test receives a foot shock S after entering the dark chamber C2.
FIG. 9 depicts a bar chart illustrating the latency to entrance into the dark chamber C2 of the rats of groups A0-A5 in trial (E).
FIG. 10 depicts a bar chart illustrating the number of BrdU-positive cells in hippocampal dentate gyrus area in trial (F).

### DETAILED DESCRIPTION OF THE INVENTION

Clavulanic acid with a chemical structure shown in FIG. 2 is a β-lactam molecule. While not effective by itself as an antibiotic, when combined with penicillin-group antibiotics, clavulanic acid can overcome antibiotic resistance in bacteria that secret β-lactamase, which otherwise inactivates most penicillin-group antibiotics.

In the present invention, clavulanic acid can be administrated to a subject in need thereof, treating seizure in the subject and/or treating epilepsy-associated motor symptom and cognitive impairment in the subject. Therefore, clavulanic acid can be used in combination with pharmaceutical acceptable vehicles, excipients, salts or other nutrients, forming a pharmaceutical composition. In addition, valproic acid can be further manufactured into any oral type that is easy to take, such as pastil, capsule, powder, pill or solution.

In the present invention, clavulanic acid can be administrated to the subject via any suitable routes. As an example, clavulanic acid can be orally or parenterally administrated to the subject, such as by intravenous injection (IV injection), intramuscular injection (IM injection), intraperitoneal injection (IP injection), transdermal administration, sublingual administration or nebulization administration.

Moreover, in the present invention, clavulanic acid can be administrated to the subject in a dosage of 0.016-10 mg/kg/day. Preferably, clavulanic acid can be administrated to the subject in the dosage of 0.016-4.99 mg/kg/day, which is lower than the clinical dosage. Clavulanic acid can be continuously or intermittently administrated to the subject. Specifically, clavulanic acid can be administered to the subject one at a predetermined interval of time. The predetermined interval of time being less than or equal to 24 hours indicates continuously administrated clavulanic acid to the subject, while the predetermined interval of time being more than 24 hours indicates intermittently administrated clavulanic acid to the subject. However, the dosage of clavulanic acid may vary according to the differences of the subject, the sequence of administration and the routes of administration, which can be appreciated by a person having ordinary skill in the art.

In addition, clavulanic acid and the conventional anticonvulsant drug (valproic acid) can be co-administrated to the subject, permitting the pharmacological effects of clavulanic acid and valproic acid overlap each other, thereby synergistically treating seizure in the subject and/or treating epilepsy-associated motor symptom and cognitive impairment in the subject. Specifically, co-administration of clavulanic acid and valproic acid includes the following ways. In the first way, clavulanic acid and valproic acid can be concurrently administrated to the subject in need thereof, which means administration of clavulanic acid and valproic acid to the subject in need thereof at the same time. In the second way, clavulanic acid and valproic acid can be sequentially administrated to the subject in need thereof, which means after administering clavulanic acid to the subject in need thereof, administering valproic acid to the subject in need thereof when the plasma drug concentration of clavulanic acid remains a therapeutic drug concentration. For example, the time interval between administering clavulanic acid and administering valproic acid is 10 minutes to 8 hours. In the third way, valproic acid and clavulanic acid are sequentially administrated to the subject in need thereof, which means after administering valproic acid to the subject in need thereof, administering clavulanic acid to the subject in need thereof when the plasma drug concentration of valproic acid remains a therapeutic drug concentration. For example, the time interval between administering valproic acid and administering clavulanic acid is 10 minutes to 8 hours. In the fourth way, clavulanic acid and valproic acid can be separately administrated to the subject in need thereof, which means after administering clavulanic acid to the subject in need thereof, administering valproic acid to the subject in need thereof when the plasma drug concentration of clavulanic acid is below the therapeutic drug concentration. For example, the time interval between administering clavulanic acid and administering valproic acid is 8-12 hours. In the fifth way, valproic acid and clavulanic acid can be separately administrated to the subject in need thereof, which means after administering valproic acid to the subject in need thereof, administering clavulanic acid to the subject in need thereof when the plasma drug concentration of valproic acid is below the therapeutic drug concentration. For example, the time interval between administering valproic acid and administering clavulanic acid is 8-12 hours.

Moreover, when valproic acid is administered together with clavulanic acid, valproic acid can be orally or parenterally administrated to the subject, such as IV injection, IM injection, IP injection, transdermal administration, sublingual administration or nebulization administration. Valproic acid can be administrated to the subject in a dosage of 0.8-30 mg/kg/day. Preferably, valproic acid can be administrated to the subject in the dosage of 0.8-19.99 mg/kg/day, which is lower than the clinical dosage. Valproic acid can be continuously or intermittently administrated to the subject. However, the dosage of valproic acid may vary according to the differences of the subject, the sequence of administration and the routes of administration, which can be appreciated by a person having ordinary skill in the art.

In addition, clavulanic acid and valproic acid can be manufactured as a pharmaceutical composition. Clavulanic acid and valproic acid can be concurrently, sequentially or separately administrated to the subject in need thereof by virtue of varying dosage form. In general, the pharmaceutical composition may include at least one pharmaceutical excipient. With such performance, the release of clavulanic acid and/or valproic acid to the subject can be controlled. As an example, liposome can be used as the pharmaceutical excipient for coating one of the active substances (clavulanic acid or valproic acid), assuring the extended releasing of the coated active substance, and therefore, the two active substances can be sequentially or separately administrated to the subject in need thereof.

In order to evaluate whether the co-administration of clavulanic acid and valproic acid can effectively treat seizure, as well as epilepsy-associated motor symptom and cognitive impairment, the following trials were carried out.

### Trial (A).

Wistar male rats (8 week-old) purchased from BioLASCO Taiwan Co., Ltd were used. The rats were housed in an animal room with constant temperature of 21-24°C, where was kept on a 12-hours light and 12-hours dark cycle. The rats were housed and kept on free diet and water.

3 days before the trials, motor functions of the rats were tested by the rotarod test. On the 1^{st}, 3^{rd}, 5^{th}, 7^{th}, 9^{th}, 11^{th} and 13^{th} days, pentylenetetrazol (PTZ, 35 mg/kg) was IP injected to the rats to induce epilepsy rats of groups A1-A5. On the 21^{st} day, after administration of PTZ, seizure intensity of the rats was measured.

Referring to TABLE 1, on the 7^{th}, 8^{th}, 9^{th}, 10^{th}, 11^{th}, 12^{th} and 13^{th} days, clavulanic acid and/or valproic acid was IP injected to the epilepsy rats of groups A2-A5. Saline (1 mL/kg/day) was IP injected to the normal rats of group A0, as well as the epilepsy rats of group A1.

**TABLE 1**

| Groups | PTZ induction epilepsy | clavulanic acid (mg/kg/day) | valproic acid (mg/kg/day) |
|---|---|---|---|
| A0 | - | 0 | 0 |
| A1 | + | 0 | 0 |
| A2 | + | 1 | 0 |
| A3 | + | 10 | 0 |
| A4 | + | 0 | 50 |
| A5 | + | 1 | 50 |

On the 14^{th} day, the motor function of the rats of groups A0-A5 was tested by rotarod test. On the 15^{th}, 16^{th} and 17^{th} days, the cognitive impairment on object recognition ability of the rats of groups A0-A5 was tested by object recognition test. On the 18^{th}, 19^{th} and 20^{th} days, the cognitive impairment on memory of the rats of groups A0-A5 was tested by passive avoidance test. Moreover, on the 22^{nd} day, BrdU (5'-bromo-2'-deoxyuridine) was IP injected to the rats of groups A0-A5 to label the proliferating cells. On the 23^{rd} day, the rats of groups A0-A5 were sacrificed and the coronal brain sections were collected. The sections with hippocampal dentate gyrus were used for BrdU staining. The number of BrdU-positive cells in hippocampal dentate gyrus was calculated.

### Trial (B).

On the 1^{st}, 3^{rd}, 5^{th}, 7^{th}, 9^{th}, 11^{th}, 13^{th} and 21^{st} days, the seizure intensity of the rats of groups A0-A5 was measured by Racine score. The seizure intensity was scored according to Racine's scale as follows: 0 = normal, nonepileptic activity, 1 = mouth and facial movements, hyperactivity, grooming, sniffing, scratching, wet dog shakes, 2 = head nodding, staring, tremor, 3 = forelimb clonus, forelimb extension, 4 = rearing, salivating, tonic clonic activity and 5 = falling, status epilepticus.

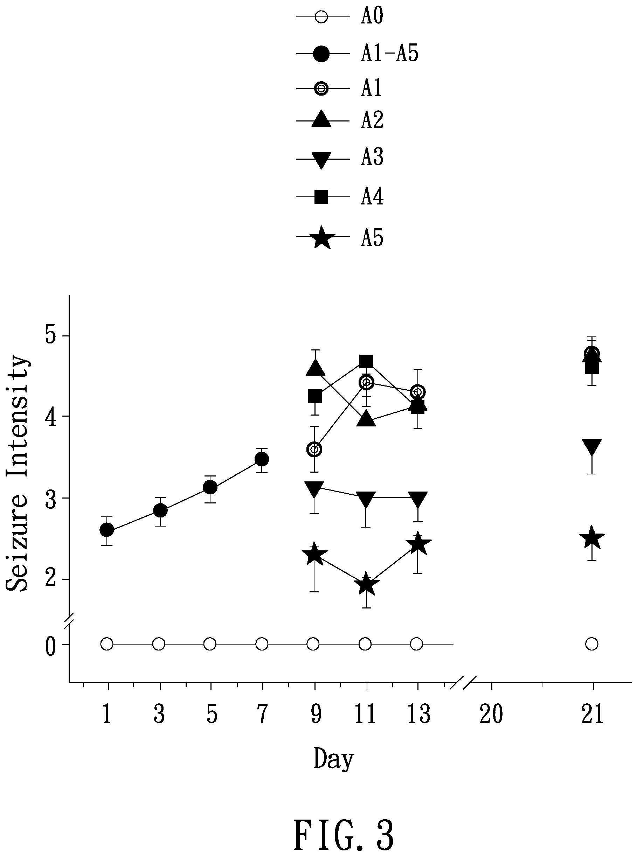

Referring to FIG. 3, the seizure intensity of the normal rats of group A0 is score 0 ("○" shown in FIG. 3). On the 1^{st}, 3^{rd}, 5^{th} and 7^{th} days, the seizure intensity of the epilepsy rats of groups A1-A5 increased ("●" shown in FIG. 3) before the epilepsy rats of groups A1-A5 were administrated by clavulanic acid and/or valproic acid. On the 9^{th}, 11^{th} and 13^{th} days, the seizure intensity of the epilepsy rats of group A1, which were administered by saline, as well as of the epilepsy rats of group A2, which were administered by low-dosage clavulanic acid, remains scores 3-4 ("⊚" and "▲" shown in FIG. 3). The seizure intensity of the epilepsy rats of group A3, which were administered by high-dosage clavulanic acid, decreases ("▼" shown in FIG. 3), indicating that the administration of high-dose clavulanic acid can effectively treat seizure. Moreover, due to the insufficient dosage of valproic acid, the seizure intensity of the epilepsy rats of group A4, which were administered by invalid dosage valproic acid, did not decrease ("■" shown in FIG. 3). In addition, the seizure intensity of the epilepsy rats of group A5, which were co-administered by low-dosage clavulanic acid and invalid dosage valproic acid, significantly decreased ("★" shown in FIG. 3), and the reduction of the seizure intensity was significantly larger than that of the epilepsy rats of group A2, which were administered by low-dosage clavulanic acid, as well as that of the epilepsy rats of group A4, which were administered by invalid dosage valproic acid, indicating that the co-administration of clavulanic acid and valproic acid shows synergistic effect on treating seizure.

Furthermore, it is worthy to be noted that after the continuously treatment for 7 days (from day 7 to day 13), even though the epilepsy rats were not treated for another 7 days (from day 14 to day 20), the seizure intensity of the epilepsy rats of group A3, which had been administered by high-dosage clavulanic acid, as well as that of the epilepsy rats of group A5, which had been co-administered by low-dosage clavulanic acid and invalid dosage valproic acid ("▼" and "★"shown in FIG. 3), was still significantly lower than the seizure intensity of the epilepsy rats of group A1, which had been administered by saline ("⊚"shown in FIG. 3). It means, effects of effective treatments were still observed after one week of drug holiday.

### Trial (C).

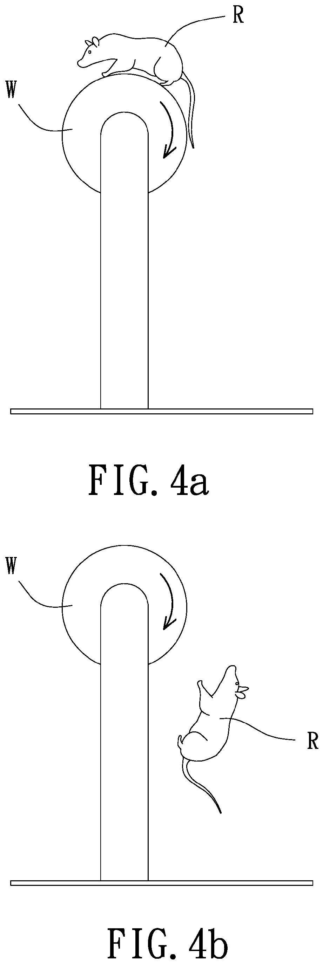

Next, motor function of the rats of groups A0-A5 was tested by rotarod test. Specifically, in the training session, the normal rat that had not yet been induced as epilepsy rat by PTZ was used as the rat R under the test. As shown in FIG. 4a, the rat R under the test rode the rotarod W at a gradually accelerated speed from 0 to 25 rpm over 6 minutes within a trial. 3 trials were performed with a cutoff of 5 minutes.

On the 14^{th} day, the rats of groups A0-A5 were used as the rats R under the test. Each of the rats R under the test was placed on the rotarod W at a constant speed of 25 rpm for 3 minutes. The faster the rat R under the test falls off the rotarod W as shown in FIG. 4b, the more severe the motor coordination and balance problems of the rat R under the test was.

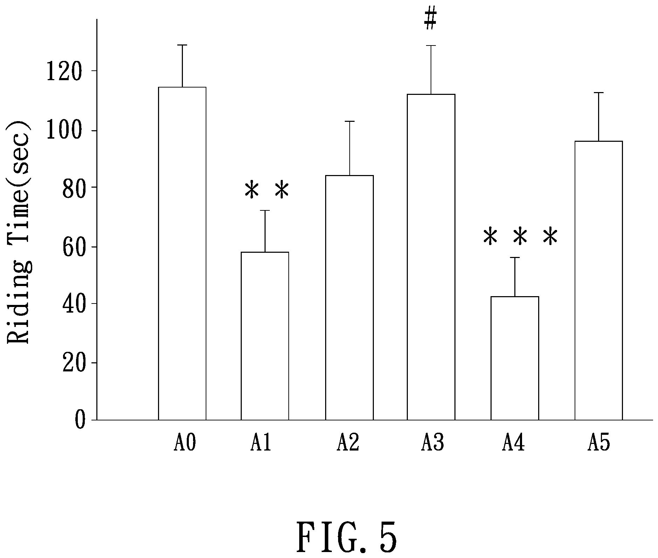

Referring to FIG. 5, compared to the normal rats of group A0, the epilepsy rats of group A1, which were administered by saline, had a significantly shorter riding time (*p*<0.01, compared to group A0). The administration of both low-dosage and high-dosage clavulanic acid can significantly increase the riding time (group A2, no difference between group A0; group A3, *p*<0.05, compared to group A1). Besides, although the administration of invalid dosage valproic acid cannot improve the motor coordination and balance problems of the epilepsy rats (group A4, *p*<0.001, compared to group A0), the co-administration of low-dosage clavulanic acid and invalid dosage valproic acid can improve the motor coordination and balance problems of the epilepsy rats (group A5, no difference between group A0).

### Trial (D).

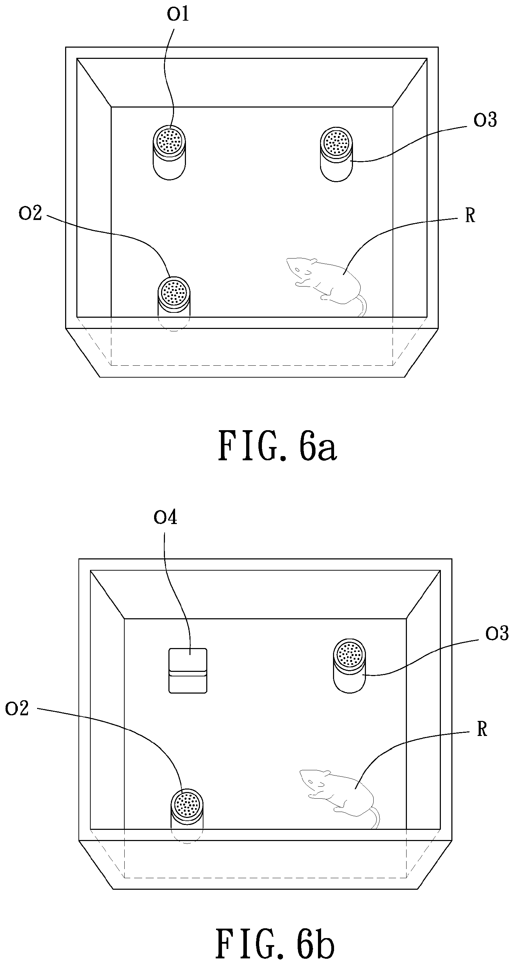

On the 15^{th}, 16^{th} and 17^{th} days, the cognitive impairment on object recognition ability of the rats of groups A0-A5 was tested by object recognition test. Specifically, during the exposure session, the rat R under the test was placed in the open box, shown as FIG. 6a, for 5 minutes. Three flavorless objects (objects O1, 02 & 03) with the same size, color, shape and material were respectively fixed at three corners of the open box.

On the 17^{th} day, during the test session, the rat R under the test was placed in the open box, shown as FIG. 6a, and the time spent exploring the object O1 (T_{O1}) and the total time spent exploring the objects O1, 02 & O3 (T_{O1+O2+O3}) were recorded, respectively. The percentage of the exploration time spent on the object O1 was calculated as (T_{O1}/T_{O1+O2+O3})^{∗}100%. After 5 minutes, a novel object O4 with different size, color, shape and material was used to replace the object O1 (shown in FIG. 6b), and the rat R under the test was placed in the open box. The time spent exploring the novel object O4 (T_{O4}) and the total time spent exploring the objects O2 & O3 and the novel object O4 (T_{O2+O3+O4}) were respectively recorded, and the percentage of the exploration time spent on the novel object O4 was calculated as (T_{O4}/T_{O2+O3+O4})^{∗}100%.

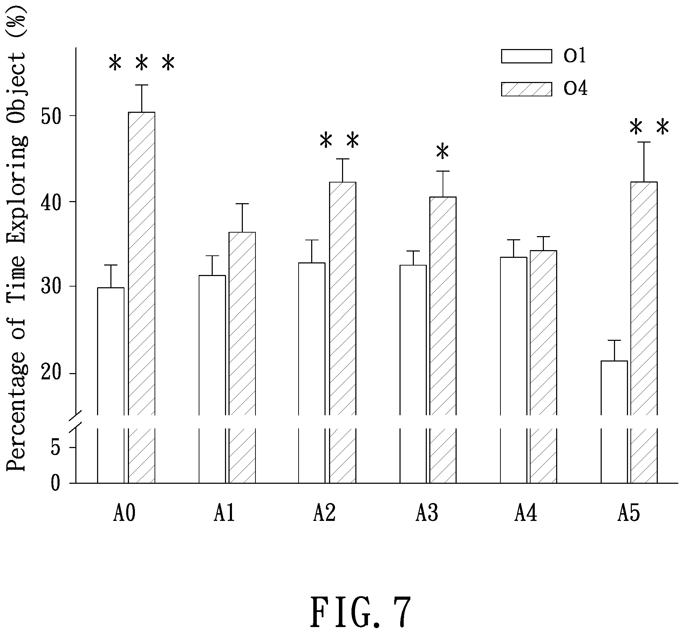

Referring to FIG. 7, the normal rats of group A0 significantly spent more time exploring the novel object O4 than exploring the object O1 (*p*<0.001), indicating that the normal rats can recognize the novel object O4 in the environment. Moreover, according to the result of the epilepsy rats of group A1, which were administered by saline, the epilepsy rats had the cognitive decline on recognition ability and cannot recognize the novel object O4. In addition, the epilepsy rats of groups A2 or A3, which were administered by low-dosage or high-dosage clavulanic acid, respectively, can recognize the novel object O4 in the environment (A2, *p*<0.01; A3, *p*<0.05). The administration of invalid dosage valproic acid cannot improve the cognitive decline on recognition ability of the epilepsy rats (group A4). It is worthy to noted that the co-administration of low-dosage clavulanic acid and invalid dosage valproic acid can improve the cognitive decline on recognition ability of the epilepsy rats (group A5, *p*=0.008), and the improvement was better than the administration of low-dosage clavulanic acid (group A2) or the administration of invalid dosage valproic acid (group A4), indication that the co-administration of clavulanic acid and valproic acid shows synergistic effect on the cognitive impairment on object recognition ability.

### Trial (E).

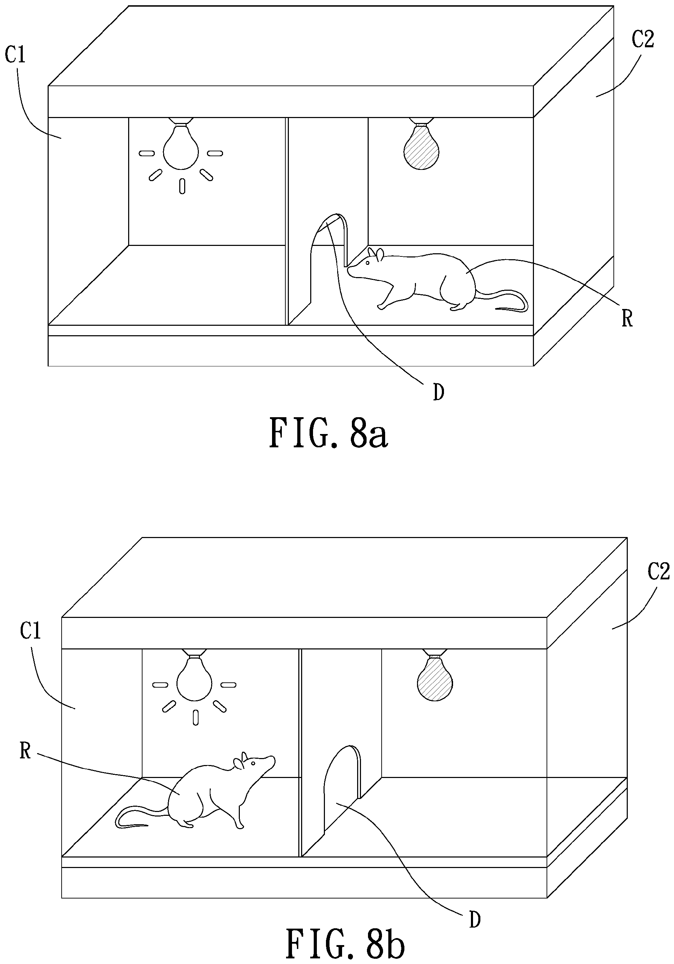

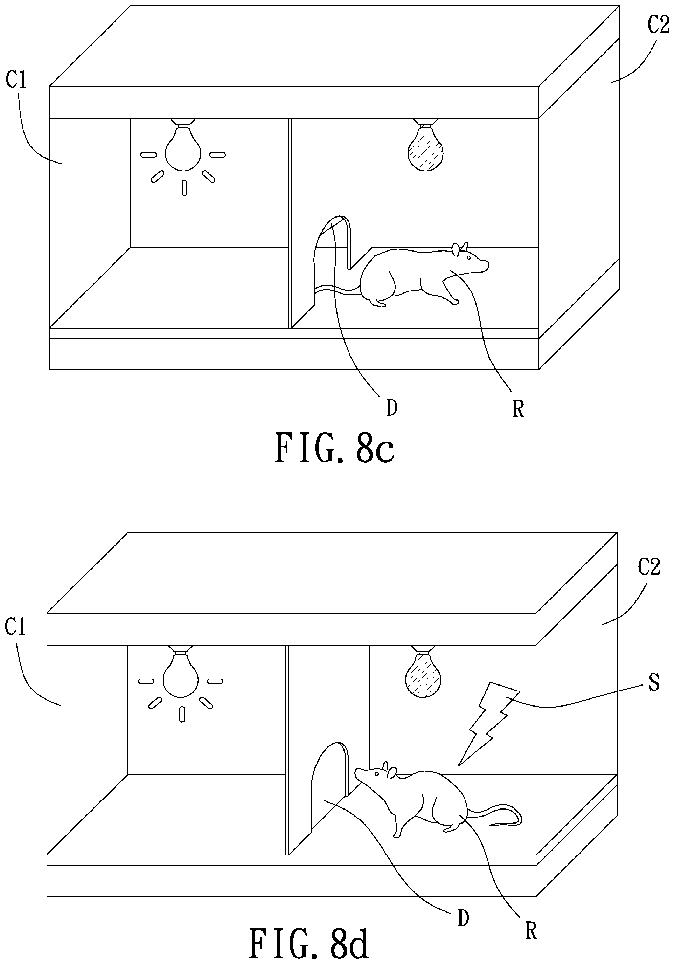

On the 18^{th}, 19^{th} and 20^{th} days, the cognitive impairment on memory of the rats of groups A0-A5 was tested by passive avoidance test using a shuttle box shown in FIGS. 8a-8d.

The shuttle box had a light chamber C1 and a dark chamber C2 divided by a guillotine door D. During the exploration session, referring to FIG. 8a, the guillotine door D was opened, and the rat R under the test was placed in the dark chamber C2. The rat R under the test was allowed to move freely into the light chamber C1.

Referring to FIG. 8b, during the learning session, the guillotine door D was closed, and the rat R under the test was placed in the light chamber C1. After 30 seconds, the guillotine door D was opened, and the rat R under the test entered the dark chamber C2 due to photophobia. Then, the guillotine door D was closed as shown in FIG. 8d, and the rat R under the test received a foot shock S after entering the dark chamber C2.

After the retention session for 24 hours, the rat R under the test was placed in the light chamber C1, and the guillotine door D was opened, as shown in FIG. 8b. The latency to entrance into the dark chamber C2 was recorded. The shorter the latency, the more severe the cognitive impairment on memory of the rat R under the test was.

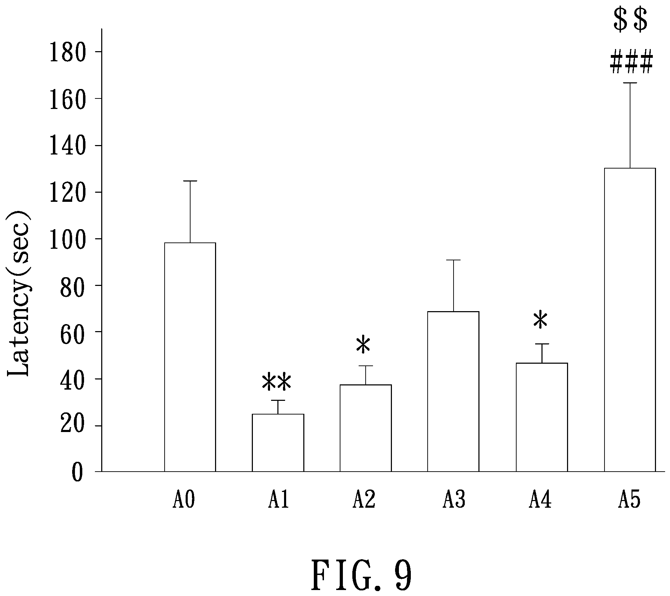

Referring to FIG. 9, compared to the normal rats of group A0, the epilepsy rats of group A1, which were administered by saline, had significantly decreased latency (*p*<0.01, compared to group A0). The latency of the epilepsy rats of group A2, which were administered by low-dosage clavulanic acid, also decreases (*p*<0.05, compared to group A0). Although the administration of invalid dosage valproic acid cannot improve the cognitive impairment on memory of the epilepsy rats (group A4, *p*<0.05, compared to group A0), the co-administration of low-dosage clavulanic acid and invalid dosage valproic acid can improve the cognitive impairment on memory of the epilepsy rats (group A5, *p*<0.001, compared to group A1; *p*<0.01, compared to groups A2 and A4), indicating that the co-administration of clavulanic acid and valproic acid shows synergistic effect on the cognitive impairment on memory.

### Trial (F).

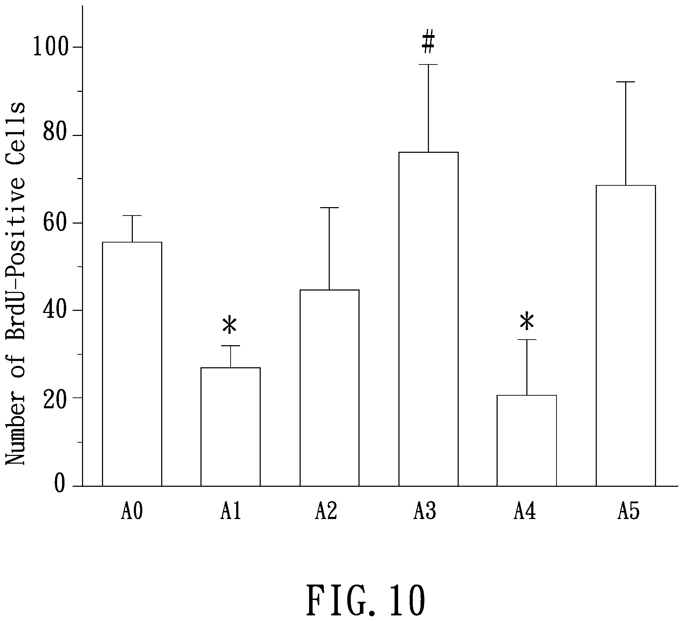

On the 22^{nd} day, BrdU was IP injected to the rats to label the proliferating cells. On the 23^{rd} day, the rats of groups A0-A5 were sacrificed and the coronal sections of the brain were collected. The sections with hippocampal dentate gyrus were used for BrdU staining. The number of BrdU-positive cells in the hippocampal dentate gyrus was calculated, as shown in FIG. 10.

Referring to FIG. 10, compared to the normal rats of group A0, the epilepsy rats of group A1, which were administered by saline, had significantly fewer BrdU-positive cells (*p*<0.05, compared to group A0), indicating that the defect on neurogenesis. The administration of invalid dosage valproic acid cannot improve the defect on neurogenesis (group A4, *p*<0.05, compared to A0). However, the administration of low-dosage or high-dosage clavulanic acid (groups A2 and A3), as well as the co-administration of low-dosage clavulanic acid and invalid dosage valproic acid (group A5), can recover the number of BrdU-positive cells to normal (no difference compared to group A0).

Besides, the aforesaid dosage can be converted into a dosage suitable for a human subject according to the dose translation formula based on body surface area (Shannon R.S. *et al.* (2007), *FASEB J.,* 22: 659-661), suggesting that 0.16-10 mg/kg/day of clavulanic acid and 0.8-30 mg/kg/day of valproic acid can be co-administrated to a human subject. Preferably, the dosage lower than the clinical dosage, that is, 0.016-4.99 mg/kg/day of clavulanic acid and 0.8-19.99 mg/kg/day of valproic acid can be co-administrated to a human subject.

Accordingly, by the synergistic effect of clavulanic acid and valproic acid, the pharmaceutical composition of the present invention can be used to prevent from seizure, to decrease seizure intensity and to trigger neurogenesis in hippocampal dentate gyrus area, treating seizure and epilepsy-associated motor symptom and cognitive impairment, such as the motor coordination and balance problems, the cognitive impairment on object recognition ability and the cognitive impairment on memory.

Moreover, the co-administration with clavulanic acid can reduce the dosage of valproic acid needed, therefore valproic acid can be rapidly and effectively metabolized by the metabolic organs such as liver and kidney, preventing valproic acid from accumulation in the organism. Furthermore, the reduced dosage of valproic acid can also decrease the burden to the metabolic organs such as liver and kidney, as well as diminish the risk of side effects.

In contrast to that conventional anticonvulsants must be continuously administered to maintain efficacy, effects of co-administration of clavulanic acid and valproic acid, suppression of epilepsy seizure and improvement of epilepsy-associated motor and cognitive deficits, are still observed after a period, for example, one week, of drug holiday. In other word, co-administration of clavulanic acid and valproic acid has a long-lasting effect.

It is worthy to be noted that the development of a method for treating a disease ultimately needs to perform clinical trials to verify the efficacy of medications on patients. In the clinical trials, in order to take into account the safety and treatment rights of the patients, one should not rashly stop the currently used medications, so the new medications that are being evaluated and the medications used had better to be subjected to an add-on study. In the present invention, the efficacy of the pharmaceutical composition including clavulanic acid and valproic acid has been verified.

## Claims

1. A pharmaceutical composition for use in the treatment of epilepsy seizure, **characterized in** the pharmaceutical composition comprises clavulanic acid and valproic acid.

2. The pharmaceutical composition for use in the treatment of epilepsy seizure according to claim 1, **characterized in that** clavulanic acid and valproic acid are to be co-administrated to a subject in need thereof to treat seizure of the subject.

3. The pharmaceutical composition for use in the treatment of epilepsy seizure according to claim 2, wherein clavulanic acid is to be administrated to the subject in a dosage of 0.016-10 mg/kg/day.

4. The pharmaceutical composition for use in the treatment of epilepsy seizure according to claim 3, wherein clavulanic acid is to be administrated to the subject in the dosage of 0.016-4.99 mg/kg/day.

5. The pharmaceutical composition for use in the treatment of epilepsy seizure according to claim 2, wherein valproic acid is to be administrated to the subject in a dosage of 0.8-30 mg/kg/day.

6. The pharmaceutical composition for use in the treatment of epilepsy seizure according to claim 5, wherein valproic acid is to be administrated to the subject in the dosage of 0.8-19.9 mg/kg/day.

7. The pharmaceutical composition for use in the treatment of epilepsy seizure according to claim 2, wherein the pharmaceutical composition is continuously to be administrated to the subject.

8. The pharmaceutical composition for use in the treatment of epilepsy seizure according to claim 2, wherein the pharmaceutical composition is intermittently to be administrated to the subject.

9. A pharmaceutical composition for use in the treatment of epilepsy-associated motor symptom and cognitive impairment, **characterized in** the pharmaceutical composition comprises clavulanic acid and valproic acid.

10. The pharmaceutical composition for use in the treatment of epilepsy-associated motor symptom and cognitive impairment according to claim 9, **characterized in that** clavulanic acid and valproic acid are to be co-administrated to a subject in need thereof to treat epilepsy-associated motor symptom and cognitive impairment of the subj ect.

11. The pharmaceutical composition for use in the treatment of epilepsy-associated motor symptom and cognitive impairment according to claim 10, wherein clavulanic acid is to be administrated to the subject in a dosage of 0.016-10 mg/kg/day.

12. The pharmaceutical composition for use in the treatment of epilepsy-associated motor symptom and cognitive impairment according to claim 11, wherein clavulanic acid is to be administrated to the subject in a dosage of 0.016-4.99 mg/kg/day.

13. The pharmaceutical composition for use in the treatment of epilepsy-associated motor symptom and cognitive impairment according to claim 10, wherein valproic acid is to be administrated to the subject in a dosage of 0.8-30 mg/kg/day.

14. The pharmaceutical composition for use in the treatment of epilepsy-associated motor symptom and cognitive impairment according to claim 13, wherein valproic acid is to be administrated to the subject in a dosage of 0.8-19.9 mg/kg/day.

15. The pharmaceutical composition for use in the treatment of epilepsy-associated motor symptom and cognitive impairment according to claim 10, wherein the pharmaceutical composition is continuously to be administrated to the subject.

16. The pharmaceutical composition for use in the treatment of epilepsy-associated motor symptom and cognitive impairment according to claim 10, wherein the pharmaceutical composition is intermittently to be administrated to the subject.

## Patentansprüche

1. Pharmazeutische Zusammensetzung zur Verwendung bei der Behandlung von epileptischen Anfällen, **dadurch gekennzeichnet, dass** die pharmazeutische Zusammensetzung Clavulansäure und Valproinsäure enthält.

2. Pharmazeutische Zusammensetzung zur Verwendung bei der Behandlung von epileptischen Anfällen gemäß Anspruch 1, **dadurch gekennzeichnet, dass** Clavulansäure und Valproinsäure einem Patienten, der sie benötigt, gemeinsam verabreicht werden, um einen Anfall des Patienten zu behandeln.

3. Pharmazeutische Zusammensetzung zur Verwendung bei der Behandlung von epileptischen Anfällen gemäß Anspruch 2, wobei Clavulansäure dem Patienten in einer Dosierung von 0,016-10 mg/kg/Tag verabreicht wird.

4. Pharmazeutische Zusammensetzung zur Verwendung bei der Behandlung von epileptischen Anfällen nach Anspruch 3, wobei Clavulansäure dem Patienten in einer Dosierung von 0,016-4,99 mg/kg/Tag verabreicht werden soll.

5. Pharmazeutische Zusammensetzung zur Verwendung bei der Behandlung von epileptischen Anfällen nach Anspruch 2, wobei Valproinsäure dem Patienten in einer Dosierung von 0,8-30 mg/kg/Tag verabreicht werden soll.

6. Pharmazeutische Zusammensetzung zur Verwendung bei der Behandlung von epileptischen Anfällen nach Anspruch 5, wobei Valproinsäure dem Patienten in einer Dosierung von 0,8-19,9 mg/kg/Tag verabreicht werden soll.

7. Pharmazeutische Zusammensetzung zur Verwendung bei der Behandlung von epileptischen Anfällen nach Anspruch 2, wobei die pharmazeutische Zusammensetzung dem Patienten kontinuierlich zu verabreichen ist.

8. Pharmazeutische Zusammensetzung zur Verwendung bei der Behandlung von epileptischen Anfällen gemäß Anspruch 2, wobei die pharmazeutische Zusammensetzung dem Patienten intermittierend verabreicht werden soll.

9. Pharmazeutische Zusammensetzung zur Verwendung bei der Behandlung von Epilepsie-assoziierten motorischen Symptomen und kognitiver Beeinträchtigung, **dadurch gekennzeichnet, dass** die pharmazeutische Zusammensetzung Clavulansäure und Valproinsäure umfasst.

10. Pharmazeutische Zusammensetzung zur Verwendung bei der Behandlung von Epilepsie-assoziierten motorischen Symptomen und kognitiver Beeinträchtigung gemäß Anspruch 9, **dadurch gekennzeichnet, dass** Clavulansäure und Valproinsäure einem Patienten, der sie benötigt, gemeinsam verabreicht werden, um Epilepsie-assoziierte motorische Symptome und kognitive Beeinträchtigung des Patienten zu behandeln.

11. Pharmazeutische Zusammensetzung zur Verwendung bei der Behandlung von Epilepsie-assoziierten motorischen Symptomen und kognitiver Beeinträchtigung gemäß Anspruch 10, wobei Clavulansäure dem Patienten in einer Dosierung von 0,016-10 mg/kg/Tag verabreicht werden soll.

12. Pharmazeutische Zusammensetzung zur Verwendung bei der Behandlung von Epilepsie-assoziierten motorischen Symptomen und kognitiver Beeinträchtigung nach Anspruch 11, wobei Clavulansäure dem Patienten in einer Dosierung von 0,016-4,99 mg/kg/Tag verabreicht werden soll.

13. Pharmazeutische Zusammensetzung zur Verwendung bei der Behandlung von Epilepsie-assoziierten motorischen Symptomen und kognitiver Beeinträchtigung nach Anspruch 10, wobei Valproinsäure dem Patienten in einer Dosierung von 0,8-30 mg/kg/Tag verabreicht werden soll.

14. Pharmazeutische Zusammensetzung zur Verwendung bei der Behandlung von Epilepsie-assoziierten motorischen Symptomen und kognitiver Beeinträchtigung nach Anspruch 13, wobei Valproinsäure dem Patienten in einer Dosierung von 0,8-19,9 mg/kg/Tag verabreicht werden soll.

15. Pharmazeutische Zusammensetzung zur Verwendung bei der Behandlung von Epilepsie-assoziierten motorischen Symptomen und kognitiver Beeinträchtigung nach Anspruch 10, wobei die pharmazeutische Zusammensetzung dem Patienten kontinuierlich verabreicht werden soll.

16. Pharmazeutische Zusammensetzung zur Verwendung bei der Behandlung von Epilepsie-assoziierten motorischen Symptomen und kognitiver Beeinträchtigung nach Anspruch 10, wobei die pharmazeutische Zusammensetzung dem Patienten intermittierend verabreicht wird.

## Revendications

1. Composition pharmaceutique destinée à être utilisée dans le traitement d'une crise d'épilepsie, **caractérisée en ce que** la composition pharmaceutique comprend de l'acide clavulanique et de l'acide valproïque.

2. Composition pharmaceutique destinée à être utilisée dans le traitement d'une crise d'épilepsie selon la revendication 1, **caractérisée en ce que** l'acide clavulanique et l'acide valproïque doivent être co-administrés à un sujet en ayant besoin pour traiter la crise du sujet.

3. Composition pharmaceutique destinée à être utilisée dans le traitement d'une crise d'épilepsie selon la revendication 2, dans laquelle l'acide clavulanique doit être administré au sujet selon une dose de 0,016 à 10 mg/kg/jour.

4. Composition pharmaceutique destinée à être utilisée dans le traitement d'une crise d'épilepsie selon la revendication 3, dans laquelle l'acide clavulanique doit être administré au sujet selon la dose de 0,016 à 4,99 mg/kg/jour.

5. Composition pharmaceutique destinée à être utilisée dans le traitement d'une crise d'épilepsie selon la revendication 2, dans laquelle l'acide valproïque doit être administré au sujet selon une dose de 0,8 à 30 mg/kg/jour.

6. Composition pharmaceutique destinée à être utilisée dans le traitement d'une crise d'épilepsie selon la revendication 5, dans laquelle l'acide valproïque doit être administré au sujet selon la dose de 0,8 à 19,9 mg/kg/jour.

7. Composition pharmaceutique destinée à être utilisée dans le traitement d'une crise d'épilepsie selon la revendication 2, dans laquelle la composition pharmaceutique doit être administrée en continu au sujet.

8. Composition pharmaceutique destinée à être utilisée dans le traitement d'une crise d'épilepsie selon la revendication 2, dans laquelle la composition pharmaceutique doit être administrée par intermittence au sujet.

9. Composition pharmaceutique destinée à être utilisée dans le traitement d'un symptôme moteur et un trouble cognitif associés à l'épilepsie, **caractérisée en ce que** la composition pharmaceutique comprend de l'acide clavulanique et de l'acide valproïque.

10. Composition pharmaceutique destinée à être utilisée dans le traitement d'un symptôme moteur et d'un trouble cognitif associés à l'épilepsie selon la revendication 9, **caractérisée en ce que** l'acide clavulanique et l'acide valproïque doivent être co-administrés à un sujet en ayant besoin pour traiter un symptôme moteur et un trouble cognitif associés à l'épilepsie du sujet.

11. Composition pharmaceutique destinée à être utilisée dans le traitement d'un symptôme moteur et d'un trouble cognitif associés à l'épilepsie selon la revendication 10, dans laquelle l'acide clavulanique doit être administré au sujet selon une dose de 0,016 à 10 mg/kg/jour.

12. Composition pharmaceutique destinée à être utilisée dans le traitement d'un symptôme moteur et d'un trouble cognitif associés à l'épilepsie selon la revendication 11, dans laquelle l'acide clavulanique doit être administré au sujet selon une dose de 0,016 à 4,99 mg/kg/jour.

13. Composition pharmaceutique destinée à être utilisée dans le traitement d'un symptôme moteur et d'un trouble cognitif associés à l'épilepsie selon la revendication 10, dans laquelle l'acide valproïque doit être administré au sujet selon une dose de 0,8 à 30 mg/kg/jour.

14. Composition pharmaceutique destinée à être utilisée dans le traitement d'un symptôme moteur et d'un trouble cognitif associés à l'épilepsie selon la revendication 13, dans laquelle l'acide valproïque doit être administré au sujet selon une dose de 0,8 à 19,9 mg/kg/jour.

15. Composition pharmaceutique destinée à être utilisée dans le traitement d'un symptôme moteur et d'un trouble cognitif associés à l'épilepsie selon la revendication 10, dans laquelle la composition pharmaceutique doit être administrée en continu au sujet.

16. Composition pharmaceutique destinée à être utilisée dans le traitement d'un symptôme moteur et d'un trouble cognitif associés à l'épilepsie selon la revendication 10, dans laquelle la composition pharmaceutique doit être administrée par intermittence au sujet.
